# EUROPÄISCHE PATENTSCHRIFT

(11) **EP 3 122 230 B1**
(45) Veröffentlichungstag und Bekanntmachung des Hinweises auf die Patenterteilung: **22.07.2020**
(21) Anmeldenummer: 15729072.7
(22) Anmeldetag: 19.02.2015
(51) Int. Cl.: A61B 1/005

(54) **ENDOSKOPISCHES INSTRUMENT**
ENDOSCOPIC INSTRUMENT
INSTRUMENT ENDOSCOPIQUE

(30) Priorität: 25.03.2014 DE 102014205556
(43) Veröffentlichungstag der Anmeldung: 01.02.2017
(73) Patentinhaber: Richard Wolf GmbH, 75438 Knittlingen (DE)
(72) Erfinder: HEIMBERGER, Rudolf, 75038 Oberderdingen (DE); BUCCHERI, Sebastian, 76448 Durmersheim (DE)
(74) Vertreter: Patentanwälte Vollmann Hemmer Lindfeld Partnerschaft mbB
(86) Internationale Anmeldenummer: PCT/DE2015/200084
(87) Internationale Veröffentlichungsnummer: WO 2015/144146

(56) Entgegenhaltungen:
- DE-A1-102005 054 057
- JP-A- 2008 099 827
- JP-A- 2011 152 361
- US-A1- 2013 226 151

## Beschreibung

Die Erfindung bettriff ein endoskopisches Instrument mit einem Schaff.

Es sind endoskopische Instrumente mit flexiblem Schaft bekannt, bei welchen ein aktiv steuerbarer Abschnitt des Schafts aus mehreren Segmenten besteht, welche aus Rohrabschnitten hergestellt sind. Diese Segmente sind durch Gelenke miteinander schwenkbeweglich verbunden. Zugmittel, welche im Inneren der Segmente geführt sind, dienen zur Steuerung der Schwenkbewegung. Für die Schwenkbewegung ist es notwendig, dass zwischen zwei benachbarten Segmenten ein ausreichend großer Spalt vorhanden ist, damit die Schwenkbewegung nicht durch gegenseitiges Berühren der Segmente auf kleine Winkel begrenzt ist. Der aktiv steuerbare Abschnitt des Schafts ist mit einem flexiblen Schlauch überzogen, welcher die im Instrument verbauten Komponenten nach außen abdichtet und eine Barriere zur Umgebung darstellt.

Ein häufiges Problem ist dabei, dass bedingt durch den Gebrauch des endoskopischen Instrumentes mit der Zeit Falten im Schlauch auftreten. Im Bereich der Spalte können so nach innen gewölbte Falten zwischen zwei benachbarte Segmente treten. Der Schlauch kann dabei zwischen den Segmenten eingeklemmt werden. Dieses kann sowohl zu einer Einschränkung der Schwenkbeweglichkeit des flexiblen Schaftes als auch zu einer Perforation des Schlauchs führen. Ansätze zum Lösen dieses Problems sind zusätzliche feine Geflechte zwischen dem flexiblen Abschnitt und dem Schlauch, ebenso wie die Nutzung eines dickwandigen Schlauchs, welcher weniger zur Faltenbildung neigt. Ebenfalls ist es bekannt, viele kurze Segmente zu verwenden, sodass die gesamte Abwinklung des endoskopischen Instruments über nur kleine Schwenkbewegungen der Segmente erreicht wird, und somit die Spalte zwischen zwei benachbarten Segmenten klein gehalten werden können. Nachteilig an diesen Lösungen ist, dass sowohl ein zusätzliches Geflecht als auch ein dickwandiger Schlauch zu einem erhöhten Außendurchmesser des endoskopischen Instruments führt, während kurze Segmente den Montageaufwand erhöhen und den Biegeradius vergrößern.

US 2013/0226151 A1 offenbart einen flexiblen Schaft für ein medizinisches Instrument, welcher aus mehreren miteinander verbundenen Schaftabschnitten gebildet ist. Dabei weisen die Schaftabschnitte an zwei diametral entgegengesetzten Seiten im Querschnitt kreisförmige Vorsprünge auf, welche in jeweils eine kreisförmige Ausnehmung eines angrenzenden Schaftabschnittes eingreifen, sodass diese um den Mittelpunkt der Kreisform verschwenkbar sind. Zwischen den so gebildeten Gelenken sind die aneinander angrenzenden Schaftabschnitte zusätzlich über rechteckige Vorsprünge oder schwalbenschwanzförmige Vorsprünge miteinander in Eingriff. Diese Ausgestaltung soll dem Schaft eine größere Stabilität geben, schränkt jedoch die Beweglichkeit des Schaftes auch ein.

DE 10 2005/054 057 A1 offenbart ebenfalls einen Schaft für ein medizinisches Instrument, welcher aus mehreren gelenkig miteinander verbundenen Schaftabschnitten gebildet ist. Dabei sind die Gelenke zwischen den Schaftabschnitten in entsprechender Weise, wie vorangehend beschrieben ausgebildet. Zwischen den Gelenken bilden sich zwischen den Schaftabschnitten Spalte, welche je nach Krümmung des Schaftes sich sehr stark erweitern, sodass Falten eines umgebenen Schlauches in die Spalte eintreten können.

Es ist Aufgabe der Erfindung, ein endoskopisches Instrument mit einem Schaft zu schaffen, bei welchem auf einfache Weise das Einklemmen eines äußeren Schlauches zwischen zwei Segmenten des Schaftes verhindert werden kann und gleichzeitig eine große Beweglichkeit des Schaftes gewährleistet werden kann.

Diese Aufgabe wird durch ein endoskopisches Instrument mit den in Anspruch 1 angegebenen Merkmalen gelöst. Bevorzugte Weiterbildungen der Erfindung ergeben sich aus den Unteransprüchen, der nachfolgenden Beschreibung und den Zeichnungen.

Das erfindungsgemäße endoskopische Instrument mit einem flexiblen Schaft bzw. einem flexiblen Schaftabschnitt weist zumindest zwei über zwei diametral entgegengesetzte Gelenke miteinander verbundene Segmente auf. Der Schaft kann entweder im Ganzen flexibel aus mehreren über Gelenke miteinander verbundenen Segmenten ausgebildet sein oder aber zumindest einen flexiblen Abschnitt aufweisen, in welchem zumindest zwei Segmente über Gelenke miteinander verbunden sind. Die Gelenke sind dabei an den bezüglich der Längsachse des Schaftes axialen Enden der Segmente angeordnet und verbinden benachbarte Segmente schwenkbeweglich miteinander. Die Gelenke sind diametral entgegengesetzt an den Segmenten so angeordnet, dass sie eine gemeinsame Drehachse aufweisen. Die Stirnkanten der Segmente werden durch die Gelenke jeweils in zwei Bereiche aufgeteilt, wobei ein Bereich jeweils durch zwei Gelenke an seinen Enden begrenzt wird und wobei beide Bereiche einer Stirnkante eines Segments vorzugsweise in Umfangsrichtung des Segments gleich lang sind. Die Gelenke sind somit bevorzugt an der Stirnkante bezogen auf den Mittelpunkt einer Umfangslinie des Segments genau gegenüberliegend angeordnet. Die axial einander zugewandten Stirnkanten der beiden verbundenen Segmente weisen zwischen sich einen Spalt auf, wobei der Spalt erfindungsgemäß zumindest einmal gewinkelt verläuft.

Somit verläuft erfindungsgemäß der Spalt in zumindest einem Teilstück mindestens einmal zur Umfangsrichtung gewinkelt, das heißt dieses Teilstück erstreckt sich im Winkel zu einer auf die Längsachse normalen Querschnittsebene des Schaftes. Die beiden Segmente, zwischen deren einander zugewandten axialen Stirnkanten der Spalt angeordnet ist, sind somit bevorzugt so gestaltet, dass eine zur Drehachse der die Segmente verbindenden Gelenke parallele Ebene den Spalt nicht durchschneiden kann, ohne gleichzeitig zumindest eine der axialen Stirnkanten der Segmente zu berühren oder zu schneiden.

Falten bilden sich zumeist in dem Teil eines den flexiblen Schaft umgebenden Schlauchs, der nahe an den Gelenken gelegen ist. Sie verlaufen zumeist entlang der Schnittlinien des Schlauchs mit den Ebenen, in welchen die Drehachse der beiden Gelenke liegt. Verläuft der Spalt nun wie erfindungsgemäß vorgesehen, so wird das Eindringen der Falten in den Spalt verhindert, da sich so der Spalt auch gewinkelt zu den sich bildenden Falten erstreckt. Alle Deformationen und insbesondere alle in Richtung des endoskopischen Instruments gerichteten Deformationen im Schlauch können ebenfalls als Falten im Sinne der Erfindung angesehen werden.

Bevorzugt weist der Spalt entlang seiner Erstreckung eine konstante Breite, vorzugsweise in axialer Richtung des Schaftes, auf. Die Breite des Spaltes ist die Ausdehnung des Spaltes zwischen zwei gegenüberliegenden axialen Stirnkanten senkrecht zur Drehachse der beiden Gelenke, welche die benachbarten Segmente verbinden. Die Breite des-Spaltes ist dabei nicht im engeren Sinn konstant. Durch die Abwinkelung zweier benachbarter Segmente ändert sich der Abstand der einander zugewandten Stirnkanten und somit auch die Breite des Spaltes abhängig vom Winkel. Als konstant ist zu verstehen, wenn die Breite des Spaltes zwischen den beiden axialen Stirnkanten entlang der Erstreckung der Stirnkanten in nicht abgewinkelter Lage der Segmente eine im Wesentlichen konstante Breite aufweist.

Die einander zugewandten axialen Stirnkanten der Segmente verlaufen bevorzugt in zumindest einem Teilbereich zwischen den Gelenken zumindest einmal achsenparallel geschwungen Der achsenparallele Verlauf einer axialen Stirnkante ist so zu verstehen, dass sie sich innerhalb oder entlang einer Umfangsfläche, die parallel zur Längsachse des Schaftes bzw. der Segmente verläuft, erstreckt. Die axiale Stirnkante verläuft dabei zumindest teilweise nicht in Umfangsrichtung zur Längsachse, sondern, wie beschrieben zumindest einmal gewinkelt zur Ausbildung des gewinkelten Spaltes.

Durch den zumindest einmal geschwungenen achsenparallelen Verlauf der einander zugewandten axialen Stirnkanten wird wie beschrieben erreicht, dass die axialen Stirnkanten der Segmente zumindest nicht vollständig in einer Ebene parallel zu der Drehachse der Gelenke verlaufen. Somit wird das Eingreifen des Schlauchs in den Spalt zwischen den Segmenten verhindert.

Aufgrund der möglichen Materialdicke und Materialform der Segmente an ihren axialen Enden wird unter einer axialen Stirnkante auch eine axiale Stirnfläche der Umfangswandung der Segmente verstanden.

Gemäß einer bevorzugten Ausführungsform der Erfindung verlaufen die einander zugewandten axialen Stirnkanten der beiden Segmente zwischen den beiden Gelenken derart wellenförmig , dass jede axiale Stirnkante zumindest einen ersten Abschnitt aufweist, welcher gegenüber zumindest einem benachbarten zweiten Abschnitt axial weiter vorsteht. Der erste Abschnitt liegt somit z. B. in Richtung der Längsachse des Schaftes weiter distalwärts als der benachbarte zweite Abschnitt derselben Stirnkante. Die Formgebung von benachbarten Stirnkanten mit ersten und zweiten Abschnitten lässt vorteilhafterweise den Spalt zwischen zwei einander zugewandten axialen Stirnkanten nicht durchgängig umfänglich zur Längsachse verlaufen, sondern gewellt oder gewinkelt. Dies verhindert das Eindringen langer Falten des Schlauchs in den Spalt zwischen den Segmenten.

Bevorzugt sind die einander zugewandten axialen Stirnkanten zweier Segmente durch die diametral entgegengesetzt angeordneten Gelenke jeweils in zwei Bereiche geteilt und beide Bereiche weisen bevorzugt jeweils zumindest einen ersten Abschnitt und einen zweiten Abschnitt auf, von welchen der erste Abschnitt gegenüber dem zweiten Abschnitt axial weiter vorsteht. Jeder Bereich entspricht einem Teil der Stirnkante, der sich entlang der Außenwandung des Segmentes zwischen den beiden anliegenden Gelenken erstreckt. Zusammen bilden beide Bereiche die gesamte Erstreckung der axialen Stirnkante an einem Axialende eines Segments.

Der erste Abschnitt einer axialen Stirnkante eines ersten Segments liegt vorzugsweise einem zweiten Abschnitt der zugewandten axialen Stirnkante eines benachbarten Segments gegenüber. Bei entsprechender Ausformung der ersten und zweiten Abschnitte ist so bevorzugt eine vor allem in axialer Richtung im Wesentlichen konstante Breite des Spaltes umsetzbar, ohne dass der Spalt sich durchgängig in Umfangsrichtung der anliegenden Segmente erstreckt. Die einander zugewandten axialen Stirnkanten von benachbarten Segmenten verlaufen bevorzugt komplementär. So kann ein erster Abschnitt der einen axialen Stirnkante in den Freiraum vor dem zweiten axialen Abschnitt der zugewandten anderen axialen Stirnkante hineinragen und beim Verschwenken sich in dem Freiraum bewegen, ohne das benachbarte Segment innerhalb eines gewissen Winkelbereichs zu berühren.

Wie oben beschrieben, teilen die Gelenke jede Stirnkante in zwei Bereiche. Die gegenüberliegenden Bereiche einander zugewandter axialer Stirnkanten zweier benachbarter Segmente sind bevorzugt ebenfalls komplementär zueinander geformt. So liegt einem ersten Abschnitt eines Bereichs ein zweiter Abschnitt des gegenüberliegenden Bereichs direkt gegenüber und umgekehrt. Wie vorhergehend beschrieben, weist der Spalt zwischen zwei Bereichen so bevorzugt eine konstante Breite entlang der Erstreckung der Bereiche auf. Besonders bevorzugt sind die vier Bereiche zweier gegenüberliegender Stirnkanten (zwei Bereiche an jeder Stirnkante) so geformt, dass der zwischen ihnen gebildete Spalt umläufig zur Längsachse eine konstante oder nahezu konstante Breite aufweist. Dies verhindert das Eindringen des Schlauchs in den Spalt und ermöglicht gleichzeitig eine gewünschte Abwinkelbarkeit. Weiterhin vorteilhaft sind alle vier Bereiche entweder identisch und/oder komplementär zueinander geformt, wobei jeweils zwei Bereiche bevorzugt komplementär zueinander geformt sind und zu jedem dieser beiden Bereiche einer der beiden übrigen Bereiche identisch geformt ist. So können beispielsweise identische Segmente um 180° gegeneinander gedreht einander gegenüberliegen.

Bei einer bevorzugten Ausführungsform der Erfindung sind die einander zugewandten axialen Stirnkanten zweier benachbarter Segmente achsenparallel S-förmig geschwungen. Eine achsenparallel S-förmig geschwungene axiale Stirnkante ist so zu verstehen, dass einer der Bereiche und vorzugsweise beide Bereiche der axialen Stirnkante jeweils zumindest einen ersten Abschnitt und einen zweiten Abschnitt aneinander angrenzend aufweisen, wobei die Abschnitte derart geformt sind, dass der jeweilige Bereich in einer Umfangsfläche um die Längsachse des Schaftes S-förmig geschwungen verläuft. Mit inbegriffen sind dabei Ausführungsformen, die einer im Profil zu erkennenden S-Form nur ähnlich oder komplementär sind. Weiterhin kann ein Bereich durch weitere sich abwechselnde erste und zweite Abschnitte gemäß vorangehender Beschreibung geformt sein und sich entlang der Umfangsfläche wellenförmig erstrecken.

Wie beschrieben ist es vorteilhaft, wenn bevorzugt die Konturen der einander zugewandten axialen Stirnkanten zweier benachbarter Segmente zumindest teilweise ineinander greifen. Wie schon aufgeführt, ergeben sich an den Axialenden jedes Segments durch die Ausformung der Stirnkanten mit ersten und zweiten Abschnitten Freiräume im Vergleich zu einer umfänglich verlaufenden axialen Stirnkante. Sind die axialen Stirnkanten zweier benachbarter Segmente so geformt, dass sie in die Freiräume der gegenüberliegenden Axialenden gegenseitig eingreifen, sodass ein Spalt zwischen den Segmenten in der Richtung parallel zur Drehachse der benachbarten Gelenke nur kleine Teilbereiche aufweist, die umfänglich zur Längsachse des Schaftes verlaufen. Das Eindringen selbst von kleinen Falten des Schlauchs in den Spalt wird so verhindert.

Gemäß einer bevorzugten Weiterbildung der Erfindung sind in achsenparalleler Ausrichtung zweier benachbarter Segmente diese derart axial zueinander beabstandet, dass die achsensenkrechte Ebene am Scheitelpunkt zumindest eines ersten Abschnittes des ersten Segments zumindest einen ersten Abschnitt des benachbarten zweiten Segments, welcher dem ersten Abschnitt des ersten Segments benachbart naheliegt, berührt oder vorzugsweise schneidet. Bei achsenparalleler, d. h. gerader Ausrichtung zweier benachbarter Segmente entlang der Längsachse des Schaftes fluchten die Längsachsen beider Segmente miteinander. Die Segmente sind somit zueinander nicht abgewinkelt. Die achsensenkrechte Ebene am Scheitelpunkt zumindest eines ersten Abschnittes entspricht einer Ebene, welche parallel zu einer Umfangslinie des Segments den ersten Abschnitt in dem Punkt berührt, welcher am weitesten von der axialen Mitte des Segments beabstandet ist, d. h. axial am weitesten vorsteht. Der Scheitelpunkt eines ersten Abschnittes einer axialen Stirnkante ist somit in axialer Richtung vorzugsweise weiter von einem Scheitelpunkt eines zweiten benachbarten Abschnitts derselben axialen Stirnkante beabstandet als der Scheitelpunkt eines ersten Abschnitts eines gegenüberliegenden Segments, der dem genannten zweiten Abschnitt gegenüberliegt.

Bei einer weiteren bevorzugten Ausführungsform sind in einer maximal zueinander gewinkelten Stellung zweier gegenüberliegender bzw. benachbarter Segmente zumindest Teilstücke der einander zugewandten axialen Stirnkanten dieser Segmente in Anlage miteinander. Es ergibt sich zumindest ein Anlagepunkt oder -bereich, in welchem sich die benachbarten Segmente berühren. Dies kann die Begrenzung der Schwenkbewegung des flexiblen Schaftes oder eines flexiblen Abschnitts des Schaftes bewirken. Ebenso gibt der gegenseitige Kontakt der benachbarten Segmente an mehreren Stellen dem Schaft Stabilität gegenüber mechanischen Kräften, welche sonst nur auf die Gelenke und/oder vorhandene Zugmittel als Kontaktpunkte zwischen den Segmenten wirken.

In einer zueinander gewinkelten Stellung benachbarter Segmente ist der Spalt in seiner Breite parallel zur Längsachse nicht mehr entlang seiner Erstreckung überall konstant oder nahezu konstant. Zwei einander zugewandte Bereiche der beiden gegenüberliegenden axialen Stirnkanten sind in einer abgewinkelten Stellung voneinander weiter beabstandet als in der nicht abgewinkelten Stellung der Segmente. Der Spalt zwischen diesen Bereichen ist verbreitert im Vergleich zu der nicht abgewinkelten Stellung. Die gegenüberliegenden Stirnkanten sind in diesem Bereich vorzugsweise so geformt, dass sie wie beschrieben erste und zweite Abschnitte aufweisen, welche auch in dieser auseinander geschwenkten Stellung noch ineinander greifen, sodass weiterhin kein durchgehender Spalt in Umfangsrichtung gebildet wird. Die anderen beiden, diametral gegenüberliegenden, einander zugewandten Bereiche sind in der abgewinkelten Stellung hingegen näher beieinander. Der Spalt ist zwischen diesen beiden Bereichen zumindest in seiner Breite verkleinert. Bevorzugt sind die einander zugewandten Stirnkanten, wie oben beschrieben, derart mit ersten Abschnitten und zweiten Abschnitten geformt, dass sie einerseits das Eindringen des Schlauchs in den Spalt in jeder abgewinkelten Stellung verhindern und andererseits die Abwinklung in dem Winkelbereich, für den das endoskopische Instrument vorgesehen ist, nicht eingeschränkt wird.

Weiterhin ist es vorteilhaft, wenn vorzugsweise zumindest zwei Segmente identisch geformt sind. Identisch geformte Segmente können somit an unterschiedlichen Stellen des flexiblen Schaftes eingesetzt werden. Besonders bevorzugt werden mehrere oder alle Segmente gleich geformt. Dies vermindert die Teilevielfalt und verringert die Kosten.

Gemäß einer bevorzugten Ausführungsform der Erfindung ist an einem Ende des distalen Segments des Schaftes ein Instrumentenkopf angegliedert. Der Instrumentenkopf bildet das distale Instrumentenende mit den Fenstern und Öffnungen für optische Systeme, Spülkanäle und Instrumente. Der Instrumentenkopf kann dabei als eigenständiges Bauteil an dem flexiblen Schaft bzw. an dem flexiblen Abschnitt angeordnet oder einstückig an das Ende eines Segments angeformt sein.

Des Weiteren ist es bevorzugt, dass Zugmittel durch zumindest Teile der Segmente geführt sind. Diese Zugmittel können beispielsweise mit dem Instrumentenkopf und/oder anderen nachfolgenden Segmenten verbunden sein und eine Steuerung der Auslenkung des flexiblen Schafts bzw. flexiblen Abschnitts des Schaftes ermöglichen. Für die Zugmittel können Führungen in den Segmenten ausgebildet sein.

Bei einer besonderen Ausführungsform sind die Zugmittel mit zumindest einem der Segmente verbunden. Dadurch ergibt sich die Möglichkeit, die Abwinklung dieses zumindest einen Segments zu steuern, indem über das Zugmittel eine Kraft auf das Segment ausgeübt wird.

Bevorzugt sind die Zugmittel so angeordnet, dass beim Abwinkeln zweier benachbarter Segmente die axialen Stirnkanten die Zugmittel nicht berühren können. Dies kann ein Verklemmen der Zugmittel und damit eine Behinderung der Steuerbarkeit der Auslenkung verhindern.

Gemäß einer weiteren vorteilhaften Ausführungsform sind die Segmente und zumindest Teile der Gelenke einstückig ausgebildet. So kann das Gelenk oder ein Gelenkteil gemeinsam mit dem Segment aus einem Werkstück einstückig gefertigt werden, so dass keine weitere Montage der Gelenkteile oder Gelenke zur Befestigung an dem Segment erforderlich ist.

Erfindungsgemäß ist vorgesehen, dass der flexible Schaft bzw. der flexible Abschnitt des Schafts zumindest abschnittsweise mit einem Schlauch ummantelt ist. Dieser an dem endoskopischen Instrument angebrachte Schlauch kann die äußere Hülle des gesamten Schaftes bilden. Der Schlauch kann aber auch nur an einem Abschnitt des Schaftes angeordnet sein. Insbesondere kann er dabei an dem flexiblen Abschnitt mit den mindestens zwei Segmenten außenumfänglich angeordnet sein. Der Schlauch dient beispielsweise dem Schutz des flexiblen Schaftes gegenüber Eindringen von Fremdkörpern. Gleichsam verhindert er, dass durch den flexiblen Schaft Gewebe verletzt wird. Durch die zuvor beschriebene Gestaltung der Segmente wird verhindert, dass dieser Schlauch in Spalte zwischen den axialen Stirnkanten der Segmente des Schaftes eindringt.

Die Erfindung ist nachfolgend anhand von in der Zeichnung dargestelltem Ausführungsbeispiel näher erläutert. Es zeigen:
- Fig. 1: eine Seitenansicht von zwei Segmenten eines flexiblen Abschnitts eines Schaftes in nicht abgewinkelter Stellung,
- Fig. 2: eine Seitenansicht der zwei Segmente nach Fig. 1,
- Fig. 3: eine vergrößerte Darstellung der Einzelheit III in Fig. 2,
- Fig. 4: eine Seitenansicht von drei Segmenten des flexiblen Abschnitts des Schaftes in einer abgewinkelten Stellung, und
- Fig. 5: eine Seitenansicht des flexiblen Abschnitts nach Fig. 4, von der Unterseite in Fig. 4 her gesehen.

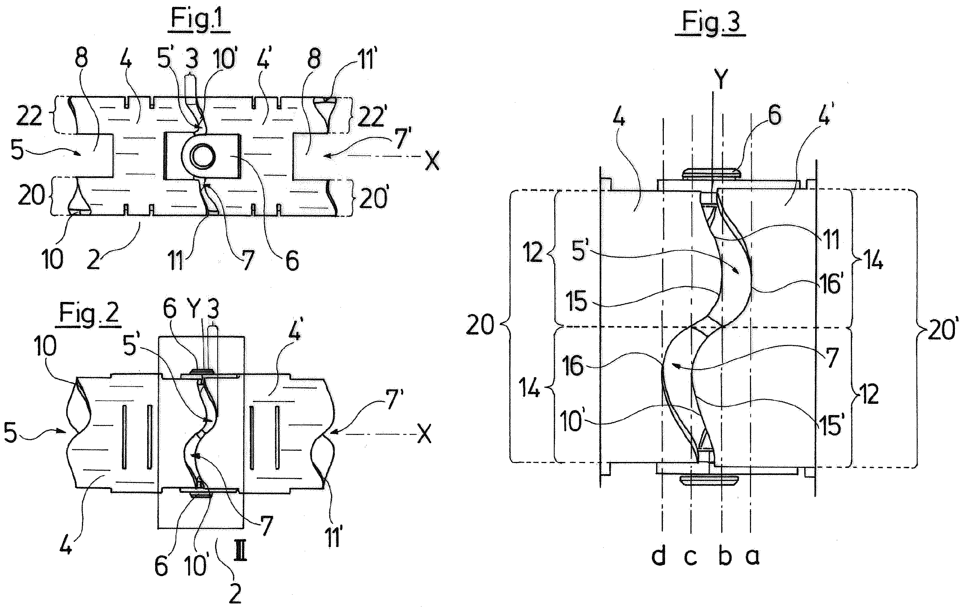

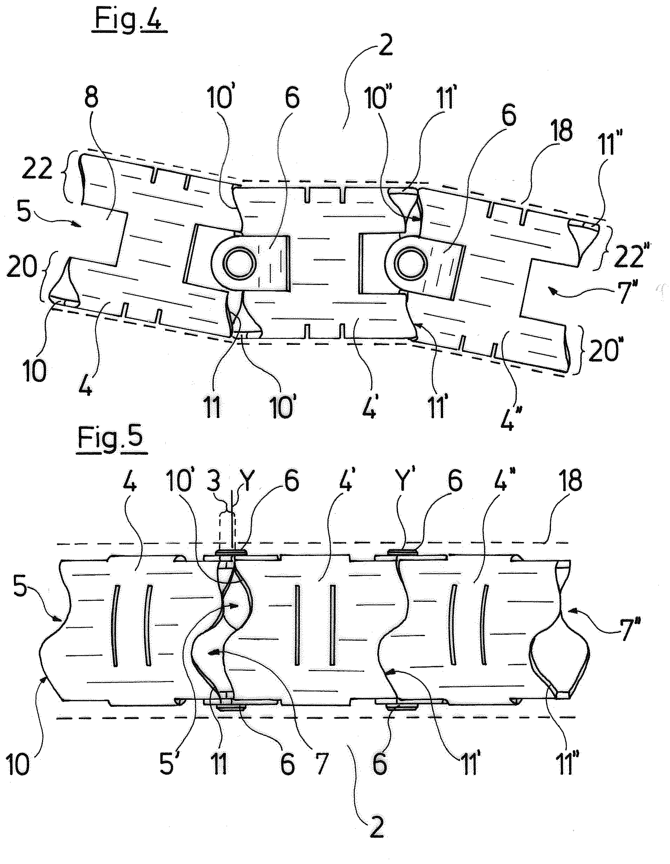

Im dargestellten Ausführungsbeispiel sind zwei Segmente 4, 4' eines flexiblen Schaftes bzw. eines flexiblen Abschnitts eines Schaftes 2 eines endoskopischen Instruments gezeigt. Die zwei Segmente 4, 4' sind identisch geformt und bilden in ihrer Grundform Hohlzylinder. Die in Fig. 1 bis 3 gezeigte nicht abgewinkelte Stellung der Segmente 4, 4' zueinander ist dadurch definiert, dass beide Segmente 4, 4' entlang der Längsachse X des Schaftes 2 ausgerichtet sind und die Segmente 4, 4' zueinander fluchten. Jedes Segment 4, 4' weist an seinem ersten Axialende 5, 5' und seinem zweiten Axialende 7, 7' jeweils eine axiale Stirnkante 10, 11, 10', 11' auf. Zwei benachbarte Segmente 4, 4' sind an ihren zugewandten Axialenden 7, 5' durch zwei als Drehgelenke ausgelegte Gelenke 6 schwenkbeweglich miteinander verbunden. Die beiden Gelenke 6 sind dabei jeweils an der Außenseite der Segmente 4, 4' in Aussparungen 8 eingelagert, liegen an dem ersten Axialende 7 des Segments 4 und an dem zweiten Axialende 5' des Segments 4' diametral entgegengesetzt und haben somit eine gemeinsame Drehachse Y (Fig. 2). In den Figuren sind die Gelenke an den Stirnkanten 10, 11' (bzw. 10, 11" in den Fig. 4 und 5) nicht eingezeichnet. Anstelle des Einlassens der kompletten Gelenke 6 in die Aussparung 8 können auch Teile der Gelenke 6 mit dem Segment 4, 4' einstückig hergestellt werden. Das vereinfacht die Montage, da die Teile der Gelenke 6 aus demselben Werkstück wie die Segmente 4, 4' geformt werden können.

Die beiden Gelenke 6 bzw. Aussparungen 8 teilen jeweils die angrenzenden axialen Stirnkanten 10, 11, 10', 11' in einen ersten Bereich 20, 20' und in einem zweiten Bereich 22, 22' auf. Die axiale Stirnkante 11 am zweiten Axialende 7 des ersten Segments 4 ist der axialen Stirnkante 10' am ersten Axialende 5' des benachbarten zweiten Segments 4' zugewandt. Zwischen den einander zugewandten axialen Stirnkanten 11, 10' der benachbarten Segmente 4, 4' befindet sich ein Spalt 3. Die axiale Stirnkante 10 bzw. 10' ist komplementär oder nahezu komplementär zur axialen Stirnkante 11 bzw. 11' geformt. Jede axiale Stirnkante 10, 10', 11, 11' weist entlang ihrer Erstreckung nebeneinander liegende erste Abschnitte 12 und zweite Abschnitte 14 auf, wobei ein erster Abschnitt 12 weiter in axialer Richtung vorsteht als zumindest ein benachbarter zweiter Abschnitt 14 derselben axialen Stirnkante 10, 10', 11, 11'. Entsprechend der zumindest nahezu komplementären Formung der axialen Stirnkante 10, 11 bzw. 10', 11' an einem Segment 4 bzw. 4' liegt bei einander zugewandten axialen Stirnkanten 11 und 10' benachbarter Segmente 4 und 4' immer ein erster Abschnitt 12 einem zweiten Abschnitt 14 gegenüber und umgekehrt.

Die axialen Stirnkanten 10, 11, 10', 11' der Segmente 4, 4' weisen innerhalb einer zur Längsachse X parallelen Umfangsfläche eine S-förmige bzw. geschwungene Form auf. Die geschwungene Form besteht aus den ersten Abschnitten 12 und zweiten Abschnitten 14, wobei sich jedem ersten Abschnitt 12 ein zweiter Abschnitt 14 (Fig. 3) entlang der axialen Stirnkante 10, 11, 10', 11' anschließt und umgekehrt. Der Übergang von einem Abschnitt zu einem anderen erfolgt stetig, so dass eine wellenförmige Kontur gebildet wird. Ein erster Bereich 20' der zum Segment 4' gehörenden axialen Stirnkante 10', welche der axialen Stirnkante 11 des Segments 4 zugewandt ist, weist in seiner Form an einem der beiden Gelenke 6 beginnend einen zweiten Abschnitt 14 auf, worauf ein erster Abschnitt 12 folgt, welcher am zweiten Gelenk 6 endet (Fig. 3). Der zweite Bereich 22' der axialen Stirnkante 10' (in Fig. 3 nicht zu erkennen) ist invers zu dem ersten Bereich 20' geformt. So weist der Bereich 22' am ersten Gelenk 6 beginnend einen ersten Abschnitt 12 auf, worauf ein zweiter Abschnitt 14 folgt, der am zweiten Gelenk 6 endet. Der zweite Bereich 22 der axialen Stirnkante 11 und der zweite Bereich 22' der axialen Stirnkante 10' sind zumindest nahezu invers oder komplementär zueinander geformt. Der erste Bereich 20 der axialen Stirnkante 11 ist zumindest nahezu invers oder komplementär zu dem ersten Bereich 20' der axialen Stirnkante 10' geformt. Weiterhin ist dabei der erste Bereich 20 der axialen Stirnkante 11 bevorzugt zumindest nahezu identisch zu dem zweiten Bereich 22' der axialen Stirnkante 10' geformt. Ebenso weisen der zweite Bereich 22 der axialen Stirnkante 11 und der erste Bereich 20 der axialen Stirnkante 10' eine zueinander zumindest nahezu identische Formung auf. An zwei gegenüberliegenden axialen Stirnkanten 10, 11' liegen sich somit bevorzugt zumindest nahezu invers oder komplementär geformte Bereiche 20, 20' bzw. 22, 22' gegenüber, wobei diametral gegenüberliegende Bereiche 20, 22' bzw. 22, 20' zumindest nahezu identisch geformt sind.

Entlang der zur Längsachse X parallelen Umfangsfläche sind somit jeweils beide Bereiche 20, 20', 22, 22' einer axialen Stirnkante 10, 11, 10', 11' wellenförmig geformt. Der Scheitelpunkt 15, 15' jedes ersten Abschnitts 12 einer Stirnkante 10, 11, 10', 11' ist der axial am weitesten vorstehende Punkt dieses ersten Abschnitts 12. Der Scheitelpunkt 16, 16' eines zweiten Abschnitts 14 einer Stirnkante 10, 11, 10', 11' ist der axial am weitesten innenliegende Punkt (der am nächsten zur axialen Mitte des Segments gelegene Punkt) dieses zweiten Abschnitts 14. Der Scheitelpunkt 15 jedes ersten Abschnitts 12 der axialen Stirnkante 11 eines Segments 4 ist in axialer Richtung X näher zum gegenüberliegenden Scheitelpunkt 16' des benachbarten Segments 4' gelegen als zumindest ein zu dem Scheitelpunkt 16' benachbarter Scheitelpunkt 15' derselben axialen Stirnkante 10'. So ist eine Tangente a am Scheitelpunkt 16' eines zweiten Abschnitts 14 an der axialen Stirnkante 10' des Segments 4' von einer Tangente b an einem dem Scheitelpunkt 16' gegenüberliegenden Scheitelpunkt 15 eines ersten Abschnitts 12 der axialen Stirnkante 11 des Segments 4 axial weniger oder gleich weit beabstandet als eine Tangente c, die an dem zu dem Scheitelpunkt 16' benachbarten Scheitelpunkt 15' eines ersten Abschnitts 12 der axialen Stirnkante 10' des Segments 4' anliegt, von der Tangente a. Ebenso gilt für die Tangente d von dem zu dem Scheitelpunkt 15 benachbarten und dem Scheitelpunkt 15' gegenüberliegenden Scheitelpunkt 16 eines zweiten Abschnitts 14 der axialen Stirnkante 11 eines Segments 4, dass der axiale Abstand der Tangente d zur Tangente c kleiner oder gleich dem axialen Abstand der Tangente d zur Tangente b ist.

Der Spalt 3 zwischen den einander zugewandten axialen Stirnkanten 11, 10' weist eine Breite auf, welche einen gewissen Wert bei zueinander fluchtender Ausrichtung der Segmente 4, 4' entlang der Längsachse X zumindest nicht unterschreitet. Dieser Wert ergibt sich aus der zu erreichenden Schwenkbarkeit der beiden Segmente 4 und 4' zueinander. Bevorzugt hat bei zueinander nicht abgewinkelter Ausrichtung der Segmente 4, 4' der Spalt 3 zumindest entlang der Erstreckung der axialen Stirnkanten 11, 10' eine im Wesentlichen konstante Breite in Richtung der Längsachse X. Die Breite des Spalts 3 ergibt sich bei komplementärer Formung der einander zugewandten axialen Stirnkanten 11, 10' beispielsweise durch den axialen Abstand der Tangenten d zu c oder b zu a.

Durch die zumindest nahezu komplementäre Formung der einander zugewandten axialen Stirnkanten 11, 10' der Segmente 4, 4' liegt immer ein erster Abschnitt 12 des Segmentes 4 einem zweiten Abschnitt 14 des Segmentes 4' gegenüber und umgekehrt. Dies gewährleistet die Schwenkbarkeit des Schaftes 2 bzw. des flexiblen Abschnitts des Schaftes 2, da der axial weiter vorstehende Abschnitt 12 in den axial zurückgezogenen Abschnitt 14 eingreift. Entlang der zur Längsachse X verlaufenden Umfangsfläche verlaufen die ersten Bereiche 20, 20' und zweiten Bereiche 22, 22' einer axialen Stirnkante 10, 11, 10', 11' ähnlich einem inversen gestreckten S bzw. einem gestreckten S.

In Fig. 4 und 5 ist am Beispiel von drei Segmenten 4, 4', 4" zu erkennen, dass ein Segment 4 bzw. 4' gegenüber einem benachbarten Segment 4' bzw. 4" sowohl rechts- als auch linksdrehend bezüglich der durch die Gelenke 6, 6' sich ergebenden Drehachsen Y, Y' abgewinkelt werden kann. Fig. 5 zeigt, wie das Segment 4 gegenüber dem Segment 4' an der gemeinsamen Drehachse Y rechtsdrehend abgewinkelt ist, während das Segment 4' selber gegenüber dem Segment 4" linksdrehend abgewinkelt ist. In der maximal abgewinkelten Stellung zweier benachbarter Segmente 4, 4' bzw. 4', 4" greifen jeweils zumindest Teile der ersten Abschnitte 12 des Segments 4 bzw. 4' an zumindest Teilen der zweiten Abschnitte 14 vorbei in das benachbarte Segment 4' bzw. 4" ein und umgekehrt, und Teile der axialen Stirnkanten 11, 10' bzw. 11', 10" der benachbarten Segmente 4, 4' bzw. 4', 4" liegen einander an und berühren sich zumindest stellenweise. Die anliegenden axialen Stirnkanten 11, 10' bzw. 11', 10" begrenzen somit das Abwinkeln der Segmente 4, 4' bzw. 4', 4" zueinander. Am Beispiel der Abwinkelung des Segmentes 4 gegenüber dem Segment 4' (Fig. 4 und 5) ist zu erkennen, dass bei maximaler rechtsdrehender Abwinkelung an der Drehachse Y sich Teile des zweiten Bereichs 22' der axialen Stirnkante 10' und Teile des zweiten Bereichs 22 der axialen Stirnkante 11 berühren. Die Berührung der beiden Bereiche kann auch entlang ihrer gesamten Erstreckung verlaufen. Ein Spalt 3 wäre somit zwischen den zweiten Bereichen 22' und 22 der axialen Stirnkante 11 nicht mehr vorhanden. Der Spalt 3 zwischen dem ersten Bereich 20' der axialen Stirnkante 10' und dem ersten Bereich 20 der axialen Stirnkante 11 hat sich bei der beispielhaften Abwinkelung verbreitert.

Die ersten Abschnitte 12 und zweiten Abschnitte 14 der beiden gegenüberliegenden ersten Bereiche 20'und 20 sind derart S-förmig oder wellenförmig ausgestaltet, dass der Spalt 3 zwischen den gegenüberliegenden ersten Bereichen 20' und 20 in ihrer maximalen Beabstandung weiterhin zumindest einmal gewinkelt verläuft. Somit ist der Abstand zwischen der Tangente b eines Scheitelpunktes 15 des ersten Bereichs 20 und der Tangente a an dem zu dem Scheitelpunkt 15 gegenüberliegenden Scheitelpunkt 16' des ersten Bereichs 20' kleiner oder gleich dem Abstand zwischen der Tangente a am genannten Scheitelpunkt 16' und der Tangente c an einem Scheitelpunkt 15' des ersten Bereichs 20', wobei der Scheitelpunkt 15' und der Scheitelpunkt 16' benachbart liegen. Ebenso ist der Abstand zwischen der Tangente d am Scheitelpunkt 16 des ersten Bereichs 20 und der Tangente c des dem Scheitelpunkt 16 gegenüberliegenden Scheitelpunktes 15' kleiner oder gleich dem Abstand zwischen Tangente d und Tangente b, wobei die Tangente b an einem zum Scheitelpunkt 16 benachbarten Scheitelpunkt 15 anliegt.

Ein Schlauch 18, welcher die Mechanik im Inneren des Schaftes 2 nach außen abdichtet, umgibt umfänglich die Segmente 4, 4', 4" eines flexiblen Schaftes 2. Der Schlauch 18 stellt eine Barriere zwischen der äußeren Umgebung und den in dem Schaft 2 befindlichen Komponenten wie bspw. Zugmitteln dar, was ein Eindringen von Gewebe oder Flüssigkeit in das Innere des Instruments verhindert. Somit ist ein Wiederaufbereiten des Instruments möglich, da nur die Außenseite des Schaftes und der Schlauch in Kontakt mit Fremdstoffen gelangen.

Die axialen Stirnkanten 10, 11, 10', 11', 10", 11" weisen bevorzugt entsprechend der vorhergehenden Beschreibung erste Abschnitte 12 und zweite Abschnitte 14 derart auf, dass ein Eindringen des Schlauchs 18 in einen Spalt 3 entlang der Erstreckung der axialen Stirnkanten 10, 11, 10', 11', 10", 11" verhindert wird.

### Bezugszeichenliste

- 2: - Teil eines flexiblen Schaftes
- 3: - Spalt
- 4, 4', 4": - Segmente
- 5, 5', 5": - erstes Axialende
- 6: - Gelenk
- 7, 7', 7": - zweites Axialende
- 8: - Aussparung
- 10, 10', 10": - Stirnkante
- 11, 11', 11": - Stirnkante
- 12: - erster Abschnitt
- 14: - zweiter Abschnitt
- 15, 15': - Scheitelpunkt eines ersten Abschnitts
- 16, 16': - Scheitelpunkt eines zweiten Abschnitts
- 18: - Schlauch
- 20, 20', 20": - erster Bereich
- 22, 22', 22": - zweiter Bereich

- X: - Längsachse der Segmente 4, 4', 4" in ungewinkelter Stellung
- Y, Y': - Drehachse diametral entgegengesetzter Gelenke 6
- a, d: - Tangente am Scheitelpunkt eines zweiten Abschnitts 14
- b, c: - Tangente am Scheitelpunkt eines ersten Abschnitts 12

## Patentansprüche

1. Endoskopisches Instrument mit einem flexiblen Schaft (2), zumindest abschnittsweise mit einem Schlauch (18) umgeben ist und welcher zumindest zwei über zwei diametral entgegengesetzte Gelenke (6) miteinander verbundene Segmente (4, 4') aufweist, zwischen deren axialen einander zugewandten Stirnkanten (11, 10') ein Spalt (3) vorhanden ist, wobei der Spalt (3) zumindest einmal gewinkelt verläuft, **dadurch gekennzeichnet, dass** die einander zugewandten axialen Stirnkanten (11, 10') der beiden Segmente (4, 4') zwischen den beiden Gelenken (6) derart achsenparallel S-förmig geschwungen verlaufen, dass jede Stirnkante (11, 10')einen ersten Abschnitt (12) aufweist, welcher gegenüber einem benachbarten zweiten Abschnitt (14) axial weiter vorsteht, und wobei der Spalt (3) zwischen den beiden axialen Stirnkanten (11, 10') in nicht abgewickelter Lage entlang seiner Erstreckung eine konstante Breite aufweist.

2. Endoskopisches Instrument nach Anspruch 1, bei welchem die einander zugewandten axialen Stirnkanten (11, 10') zweier Segmente (4, 4') durch die diametral entgegengesetzt angeordneten Gelenke (6) in zwei Bereiche geteilt sind und beide Bereiche jeweils einen ersten Abschnitt (12) und einen zweiten Abschnitt (14) aufweisen, von welchen der erste Abschnitt (12) gegenüber dem zweiten Abschnitt (14) axial weiter vorsteht.

3. Endoskopisches Instrument nach Anspruch 1 oder 2, bei welchem der erste Abschnitt (12) der axialen Stirnkante (11) eines ersten Segments (4) dem zweiten Abschnitt (14) der gegenüberliegenden axialen Stirnkante (10') des benachbarten Segments (4') gegenüberliegt.

4. Endoskopisches Instrument nach einem der vorangehenden Ansprüche, bei welchem die Konturen der einander zugewandten axialen Stirnkanten (11, 10') zweier benachbarter Segmente (4, 4') zumindest teilweise ineinander greifen.

5. Endoskopisches Instrument nach einem der vorangehenden Ansprüche , bei welchem in achsenparalleler Ausrichtung (X) zweier benachbarter Segmente (4, 4') diese derart axial zueinander beabstandet sind, dass die achsensenkrechte Ebene am Scheitelpunkt (15) zumindest eines ersten Abschnitts (12) des ersten Segments (4) zumindest einen ersten Abschnitt (12) des zugewandten zweiten Segments (4'), welcher dem ersten Abschnitt (12) des ersten Segments (4) benachbart nahe liegt, berührt oder vorzugsweise schneidet.

6. Endoskopisches Instrument nach einem der vorangehenden Ansprüche, bei welchem in einer maximal zueinander gewinkelten Position zumindest Teilbereiche der einander zugewandten axialen Stirnkanten (11, 10') zweier benachbarter Segmente (4, 4') in Anlage miteinander sind.

7. Endoskopisches Instrument nach einem der vorangehenden Ansprüche, bei welchem zumindest zwei Segmente (4, 4') identisch geformt sind.

8. Endoskopisches Instrument nach einem der vorangehenden Ansprüche, bei welchem an einem Ende eines Segments (4, 4') ein Instrumentenkopf angegliedert ist.

9. Endoskopisches Instrument nach einem der vorangehenden Ansprüche, bei welchem Zugmittel durch zumindest Teile der Segmente (4, 4', 4") geführt sind.

10. Endoskopisches Instrument nach Anspruch 8, bei welchem die Zugmittel mit zumindest einem der Segmente (4, 4', 4") verbunden sind.

11. Endoskopisches Instrument nach einem der vorangehenden Ansprüche, bei welchem die Segmente (4, 4', 4") und zumindest Teile der Gelenke (6) einstückig ausgebildet sind.

## Claims

1. An endoscopic instrument with a flexible shank (2) which at least in sections is surrounded by a flexible tube (18) and which comprises at least two segments (4, 4') which are connected to one another via two diametrically opposed joints (6) and between whose axial face edges (11, 10') which face one another a gap (3) is present, wherein the gap (3) runs in an angled manner at least once, **characterised in that** the axial face edges (11, 10') of the two segments (4, 4') which face one another, between the two joints (6) run in an S-shaped curved manner parallel to the axis such that each face edge (11, 10') comprises a first section (12) which projects axially further compared to an adjacent second section (14), and wherein the gap (3) between the two axial face edges (11, 10') has a constant width along its extension in the non-angled position.

2. An endoscopic instrument according to claim 1, with which the axial face edges (11, 10') of two segments (4, 4') which face one another are divided by the diametrically oppositely arranged joints (6) into two regions, and both regions each comprise a first section (12) and a second section (14), of which the first section (12) projects further axially compared to the second section (14).

3. An endoscopic instrument according to claim 1 or 2, with which the first section (12) of the axial face edge (11) of a first segment (4) lies opposite the second section (14) of the opposite axial face edge (10') of the adjacent segment (4').

4. An endoscopic instrument according to one of the preceding claims, with which the contours of the axial face edges (11, 10') of two adjacent segments (4, 4'), said face edges facing one another, at least partly engage into one another.

5. An endoscopic instrument according to one of the preceding claims, with which two adjacent segments (4, 4') in their axis-parallel alignment (X) are axially distanced to one another in a manner such that the plane perpendicular to the axis and on the apex (15) of at least a first section (12) of the first segment (4) contacts or preferably intersects at least a first section (12) of the facing second segment (4') which lies closely adjacent the first section (12) of the first segment (4).

6. An endoscopic instrument according to one of the preceding claims, with which in a position of being maximally angled to one another, at least part-regions of the axial face edges (11, 10') of two adjacent segments (4, 4'), said face edges facing one another, are in contact with one another.

7. An endoscopic instrument according to one of the preceding claims, with which at least two segments (4, 4') are identically shaped.

8. An endoscopic instrument according to one of the preceding claims, with which an instrument head is integrated at one end of a segment (4, 4').

9. An endoscopic instrument according to one of the preceding claims, with which pull means are led through at least parts of the segments (4, 4', 4")

10. An endoscopic instrument according to claim 8, with which the pull means are connected to at least one of the segments (4, 4', 4").

11. An endoscopic instrument according to one of the preceding claims, with which the segments (4, 4', 4") and at least parts of the joints (6) are designed as one piece.

## Revendications

1. Instrument endoscopique doté d'une tige (2) flexible, entourée au moins en partie d'un flexible (18) et qui comporte au moins deux segments (4, 4') reliés entre eux par l'intermédiaire de deux articulations (6) diamétralement opposées, une fente (3) étant présente entre leurs bords frontaux axiaux (11, 10') se faisant face, la fente (3) étant coudée au moins une fois, **caractérisé en ce que** les bords frontaux axiaux (11, 10') se faisant face des deux segments (4, 4') entre les deux articulations (6) sont incurvés en forme de S d'une manière parallèle à l'axe de façon telle que chaque bord frontal (11, 10') présente une première partie (12) qui fait davantage saillie axialement par rapport à une seconde partie (14) adjacente, et la fente (3) entre les deux bords frontaux axiaux (11, 10') présente une largeur constante en position non développée le long de son étendue.

2. Instrument endoscopique selon la revendication 1, dans lequel les bords frontaux axiaux (11, 10') se faisant face de deux segments (4, 4') sont divisés en deux zones par les articulations (6) disposées de façon diamétralement opposée et les deux zones présentent chacune une première partie (12) et une seconde partie (14), la première partie (12) faisant davantage saillie axialement par rapport à la seconde partie (14).

3. Instrument endoscopique selon la revendication 1 ou 2, dans lequel la première partie (12) du bord frontal axial (11) d'un premier segment (4) fait face à la seconde partie (14) du bord frontal axial (10') opposé du segment adjacent (4').

4. Instrument endoscopique selon l'une des revendications précédentes, dans lequel les contours des bords frontaux axiaux (11, 10') se faisant face de deux segments (4, 4') adjacents s'imbriquent au moins partiellement.

5. Instrument endoscopique selon l'une des revendications précédentes, dans lequel, dans l'alignement (X) parallèle à l'axe de deux segments (4, 4') adjacents, ceux-ci sont axialement espacés l'un de l'autre de façon telle que le plan perpendiculaire à l'axe, au sommet (15) d'au moins une première partie (12) du premier segment (4), touche ou de préférence coupe au moins une première partie (12) du second segment (4') lui faisant face, lequel est adjacent à la première partie (12) du premier segment (4).

6. Instrument endoscopique selon l'une des revendications précédentes, dans lequel, dans une position coudée au maximum d'un segment par rapport à l'autre, au moins des régions partielles des bords frontaux axiaux (11, 10') se faisant face de deux segments (4, 4') adjacents sont en contact mutuel.

7. Instrument endoscopique selon l'une des revendications précédentes, dans lequel au moins deux segments (4, 4') sont de configuration identique.

8. Instrument endoscopique selon l'une des revendications précédentes, dans lequel une tête d'instrument est rattachée à une extrémité d'un segment (4, 4').

9. Instrument endoscopique selon l'une des revendications précédentes, dans lequel des moyens de traction sont guidés à travers au moins des parties des segments (4, 4', 4").

10. Instrument endoscopique selon la revendication 8, dans lequel les moyens de traction sont reliés à au moins l'un des segments (4, 4', 4").

11. Instrument endoscopique selon l'une des revendications précédentes, dans lequel les segments (4, 4', 4") et au moins des parties des articulations (6) sont réalisés d'une seule pièce.
